# EUROPÄISCHE PATENTSCHRIFT

(11) **EP 1 991 875 B1**
(45) Veröffentlichungstag und Bekanntmachung des Hinweises auf die Patenterteilung: **23.09.2009**
(21) Anmeldenummer: 07723032.4
(22) Anmeldetag: 03.03.2007
(51) Int. Cl.: G01N 33/68

(54) **TEST ZUM NACHWEIS VON PATHOLOGISCHEN PRIONEN**
TEST FOR THE DETECTION OF PATHOLOGICAL PRIONS
TEST POUR DÉCELER DES PRIONS PATHOLOGIQUES

(30) Priorität: 06.03.2006 DE 102006010647
(43) Veröffentlichungstag der Anmeldung: 19.11.2008
(73) Patentinhaber: Latza, Reinhard, Dr., 66386 St. Ingbert (DE)
(72) Erfinder: Latza, Reinhard, Dr., 66386 St. Ingbert (DE)
(74) Vertreter: Luderschmidt, Schüler & Partner
(86) Internationale Anmeldenummer: PCT/EP2007/001844
(87) Internationale Veröffentlichungsnummer: WO 2007/101631

(56) Entgegenhaltungen:
- WO-A-03/073106
- XANTHOPOULOS ET AL: "Tissue plasminogen activator in brain tissues infected with transmissible spongiform encephalopathies" NEUROBIOLOGY OF DISEASE, BLACKWELL SCIENCE, OXFORD, GB, Bd. 20, Nr. 2, November 2005 (2005-11), Seiten 519-527, XP005118686 ISSN: 0969-9961
- DATABASE WPI Week 200401 Derwent Publications Ltd., London, GB; AN 2004-003614 XP002439411 & JP 2003 321498 A (FUJIREBIO KK) 11. November 2003 (2003-11-11)

## Beschreibung

Die vorliegende Erfindung betrifft ein Verfahren zum Nachweis von pathologischen Prionproteinen in vitro in einer Probe und einen diagnostischen Kit zur Durchführung dieses Verfahrens.

Die übertragbaren spongiformen Enzephalopathien (transmissiblen spongiformen Enzephalopathien, TSE) oder Prionkrankheiten sind degenerative Himerkrankungen, die mit charakteristischen schwammartigen histologischen Veränderungen des Gehirns einhergehen und stets tödlich verlaufen. Nach der Theorie von Prusiner (Science 1982, 216: 136-144) ist das Agens dieser Erkrankungen ein infektiöses Protein ohne nachweisbare Nukleinsäure, das Prion ("proteinaceous infectious agent"). Es handelt sich dabei um eine fehlgefaltete Form (PrP^{Sc}, Sc: "Scrapie") eines natürlicherweise vorkommenden Proteins, des zellulären Prionproteins (PrP^{C}). Die Vermehrung des Erregers erfolgt durch Umwandlung der normalen Struktur des Prionproteins in die fehlgefaltete Form, deren Auftreten mit der Infektion bzw. mit der Erkrankung assoziiert ist. In Übereinstimmung mit dieser Hypothese sind Mausstämme, denen das Prionprotein fehlt, experimentell nicht infizierbar. Demzufolge werden die TSE-Erreger auch häufig als Prionen bezeichnet und der gesamte Formenkreis dieser Krankheitsbilder als Prion-Erkrankungen zusammengefasst.
Die spongiformen Enzephalopathien kommen bei vielen Säugetieren einschließlich des Menschen vor. Beim Menschen handelt es sich um die Creutzfeldt-Jakob-Krankheit (CJK), das Gerstmann-Sträussler-Scheinker-Syndrom (GSS), die letale familiäre Insomnie (FFI), Kuru und die Variante der Creutzfeldt-Jakob-Krankheit (vCJK). Am längsten ist die Traberkrankheit (Scrapie) bei Schafen bekannt. Seit 1984 ist die bovine spongiforme Enzephalopathie (BSE) und seit 1996 die Variante der Creutzfeldt-Jakob-Krankheit dokumentiert. Seitdem sind in Großbritannien und anderen EU-Länder über 180.000 Rinder an der bovinen spongiformen Enzephalopathie (BSE) erkrankt und getötet worden. In Deutschland ist bei mehr als 290 Rindern die BSE nachgewiesen worden.
1993 erkrankten erstmals 2 junge britische Bauern an einer ungewöhnlichen Form der Creutzfeldt-Jakob-Krankheit (CJK), die 1996 als die neue Variante der CJK (vCJK) beschrieben wurde. Bis dato sind dieser Erkrankung über 100 Menschen in Großbritannien anheimgefallen. Heute kann als gesichert gelten, dass es sich dabei um BSE beim Menschen handelt.

Angesichts des tödlichen Verlaufs, der Übertragbarkeit auf Menschen, der langen Inkubationszeiten und der fehlenden Therapien ist die Diagnostik der transmissiblen spongiformen Enzephalopathien von größter Bedeutung.

Bisher haben drei BSE-Schnelltests die EU-Zulassung erhalten (EUROPEAN COMMISSION (1999) DIRECTORATE-GENERAL XXIV CONSUMER POLICY AND CONSUMER HEALTH PROTECTION Directorate B - Scientific Health Opinions The Evaluation of Tests for the Diagnosis of transmissible spongioform encephalopathy in bovines www.eu-komission.de):
- Prionics Check, ursprünglich entwickelt von der Firma Prionics AG (Zürich, Schweiz), wird seit dem 1. Februar 2001 von Roche Diagnostics weltweit vermarktet. Der Test basiert auf dem Western-Blot, die Testdauer beträgt sieben bis acht Stunden.
- Platelia^{®} BSE-Test wird von der Firma Bio-Rad Laboratories (USA) vertrieben und wurde zusammen mit der Commission de L'Energie Atomique (Frankreich) entwickelt. Zugrunde liegt ein ELISA; die Testdauer beträgt vier bis sieben Stunden.
- Enfer TSE der Firma Enfer Technology (Irland) basiert auf dem ELISA-Prinzip; die Testdauer beträgt vier Stunden.

Alle drei Tests verwenden Prion-spezifische Antikörper gegen das Bruchstück PrP²⁷⁻³⁰. Werden PrP^{C} und Pr^{Sc} mit dem proteolytischen Enzym Proteinase K behandelt, wird PrP^{C} vollständig verdaut, während Pr^{Sc} auf Grund seiner strukturellen Verschiedenheit nur partiell verdaut wird. Es verbleibt das Proteinase-K-resistente Fragment PrP²⁷⁻³⁰, das anschließend nachgewiesen wird. Die Verdauung durch Proteinase K erfordert zusätzliche Arbeitsschritte in diesen Tests und exakte Kontrolle der Konzentration und Wirkungszeit des Enzyms, so dass nach verlängerter Behandlung auch die pathologischen Prionen fast vollständig verdaut werden können. Da die Verdauung zuerst in der Probe erfolgt und danach die Prionen spezifisch nachgewiesen werden, verliert diese Methode an Empfindlichkeit. Eine weitere Gemeinsamkeit: Die Tests können erst post mortem durchgeführt werden und benötigen weniger als ein Gramm Gewebe aus dem Stammhirn, in dem besonders viele PrP^{Sc} -Moleküle akkumuliert werden. Ein Nachteil aller drei Tests ist die nicht ausreichende Empfindlichkeit. Die Krankheit muss sich in einem fortgeschrittenen Stadium mit entsprechend starker Anhäufung von BSE-Prionen befinden, damit man klare Testergebnisse erhält. Deshalb wenden offizielle Behörden und Institute in Verdachtsfällen oder zur Absicherung einer Diagnose andere Methoden wie Histopathologie und Immunhistochemie an. Neue Techniken wie Immuno-PCR, spezifische Liganden-Adsorption und Fluoreszenz-Korrelations-Spektroskopie (FCS) werden erforscht, um die Testempfindlichkeit zu verbessern.

Eine weitere Methode, der konforrnationsabhängige Assay (conformation dependent immunoassay-CDI; Safar J. et al., Nature Medicine 1998, 4: 10, 1157-1165), basiert auf der spezifischen Konformation des PrP^{Sc}-Moleküls und zwar auf der teilweise verdeckten Bindungsstelle für den monoklonalen Antikörper 3F4. Zum Nachweis der pathologischen Form wird das Verhältnis des Signals zwischen nativer und denaturierter (entfaltetes PrP-Molekül) Probe verwendet. Diese Methode erfordert auch eine zusätzliche Vorbehandlung der Probe und ist relativ zeitaufwändig.

Eine andere Reihe von Nachweismethoden verwendet Techniken zum Anreichern der Probe mit pathologischen Prionen. Eine Methode dieser Reihe ist die PMCA-(protein misfolding cyclic amplification) Methode von Soto (Fa. Serono; Castilla J. et al. Nature Medicine Online Publication 28.08.2005). Hierzu wird PrP^{Sc} im Überschuss von PrP^{C} inkubiert, um die PrP^{Sc}-Aggregate zu vermehren, die bei der nachfolgenden Ultraschallbehandlung zerstört werden, so dass neue, kleinere Aggregate gebildet werden. Letztere dienen als "Matrize" zur Bildung neuerer PrP^{Sc}-Aggregate.
Diese Zyklen werden mehrmals wiederholt (bis zu 150 mal). Bei der PMCA-Methode vergehen mindestens 75 Stunden bis die berichtete Sensitivität erreicht wird. Zudem wird als "Matrize" Hamster-Hirngewebe zugefügt und es ist unklar, in welchem Stadium der Infektion sich die untersuchten Tiere befanden.

Die Serinprotease Plasmin (bevorzugte Spaltstelle Lys-Xaa>Arg-Xaa) ist ein von Plasminogen, einem ubiquitären Zymogen-Präkursor; synthetisiertes Enzym, das eine wichtige Rolle bei der Umwandlung des Fibrins in lösliche Produkte (Fibrinolyse) und bei dem proteolytischen Abbau der extrazellulären Matrix (plasmininduzierte Proteolyse) spielt. In letzter Zeit wurde berichtet, dass Plasmin in der Lage ist, PrP^{C} in vitro zu spalten, und dass PrP^{C} und die NH₂-Region des PrP-Moleküls die t-PA (tissue-type plasminogen activator) vermittelte Plasminbildung stimulieren können. Es wurde weiterhin gefunden, dass die Primärspaltstelle des Plasmins auf dem PrP-Molekül in der Region der Aminosäurereste 108-112 liegt. Über die Aktivität von Plasmin gegenüber der pathologischen Form (PrP^{Sc}) des Prionproteins liegen allerdings noch keine weiteren Erkenntnisse vor.

Somit gibt es bisher keine routinemäßig einsetzbaren Testverfahren, mit denen eine eindeutige frühe Diagnose am lebenden Tier oder am Menschen während der Inkubationszeit, d. h. vor Eintritt von klinisch erfassbaren Symptomen, gestellt werden kann.

Es besteht daher Bedarf an einem Schnelltest zum Nachweis von pathologischen Prionen, der die vorstehend genannten Nachteile des Stands der Technik überwindet.

Der vorliegenden Erfindung lag daher die Aufgabe zugrunde, ein Test zum Nachweis von pathologischen Prionen zur Verfügung zu stellen, der eine hohe Sensitivität aufweist, der mit geringem Zeitaufwand und vergleichsweise geringen Kosten gegebenenfalls automatisiert durchgeführt werden kann, der in der Lage ist, pathologische Prionen in einem frühen Krankheitsstadium nachzuweisen, und bei dem auf eine Proteinase K-Behandlung verzichtet werden kann.

Diese und andere für den Fachmann ohne weiteres ersichtliche Aufgaben werden durch die nachstehend beschriebene Erfindung gelöst.

Überraschenderweise wurde gefunden, dass sich die pathologische Form des Prionproteins mit hoher Selektivität und relativ geringem Aufwand in vitro in einer Probe nachweisen lässt, wenn man:
a) Capture-Antikörper, die sowohl die pathologische (PrP^{Sc}) als auch die nicht-pathologische Form (PrP^{C}) des Prionproteins erkennen, auf einer festen Phase fixiert;
b) die Probe mit den fixierten Antikörpern aus Schritt a) inkubiert, wobei die pathologische und die nicht-pathologische Form des Prionproteins an die fixierten Antikörper unter Bildung von Komplexen binden;
c) die Probe von den entstandenen Komplexen abtrennt;
d) die Komplexe mit Plasmin inkubiert, wobei die nicht-pathologische Form des Prionproteins gespalten wird;
e) die durch die Inkubation mit Plasmin erhaltenen Spaltfragmente von den Komplexen abtrennt; und
f) das in den Komplexen enthaltene, nicht gespaltene Prionprotein mit Detektions-Antikörpern detektiert.

Durch die Behandlung mit Plasmin wird überraschenderweise die nicht-pathologische Form des Prionproteins gespalten, während die pathologische Form - des Prionproteins unverdaut bleibt. Die pathologische Form hat die gleiche Aminosäuresequenz wie die physiologische Form des Prionproteins, aber eine davon verschiedene Raumstruktur. Es wurde gefunden, dass die Primärspaltstelle für Plasmin in allen untersuchten Spezies im Bereich der Aminosäurereste 106 bis 126 des Prionproteins liegt. Die Primärspaltstelle der pathologischen Form liegt teilweise verdeckt, getarnt ("buried core") und ist somit schwer zugänglich für die enzymatische Aktivität des Plasmins. Die gute Spaltbarkeit der physiologischen Form im Vergleich zu der schlechten Spaltbarkeit der pathologischen Form ist das Prinzip der hier entwickelten Methode zum Unterscheiden zwischen den beiden Prionenformen.

Erfindungsgemäß werden mit diesem Verfahren pathologische Prionproteine nachgewiesen.

Hierin verwendet bedeuten:
- PrP:: das Prionprotein allgemein, zum Beispiel wenn auf strukturelle Charakteristika des Prionproteins bezug genommen wird;
- PrP^{C}:: die zelluläre Form des Prionproteins, also dessen in gesunden Zellen vorliegende, nicht pathologische Form; und
- PrP^{Sc}:: die pathologische Form des Prionproteins.

Die für das Verfahren entnommene Probe kann dabei grundsätzlich von jedem menschlichen oder tierischen Subjekt stammen, das im Verdacht steht, die pathologische Form des Prionproteins aufzuweisen. Die Probe kann zum Beispiel menschlichen Ursprungs sein, oder von einem Rind oder einem Hamster stammen. Die Probe kann einem lebenden oder toten Subjekt entnommen werden. Grundsätzlich kann als Ausgangsmaterial für die Probe jedes flüssige oder feste, von dem Körper des Subjektes stammende Material dienen, das die pathologische Form des Prionproteins enthalten könnte. Beispielhafte Ausgangsmaterialien für die Proben können Blutproben, Gewebeproben oder Körperflüssigkeiten wie Urin, Milch, Liquor oder Speichel sein. Vor allem bei festen Proben kann es notwendig sein, das Ausgangsmaterial zunächst aufzuschließen, so dass die pathologische Form des Prionproteins für das erfindungsgemäße Verfahren in geeigneter Form vorliegt. Diese Aufschlussverfahren sind dem Fachmann seit langem wohlbekannt.

Bei dem erfindungsgemäßen Verfahren wird zunächst ein Capture-Antikörper auf einer festen Phase fixiert.

Der Capture-Antikörper weist die Eigenschaft auf, sowohl die pathologische Form (PrP^{Sc}) als auch die nicht-pathologische Form des Prionproteins (PrP^{C}) zu erkennen und zu binden. Die Capture-Antikörper können zum Beispiel monoklonal oder polyklonal sein. Geeignete Capture-Antikörper können nach Standardmethoden selbst hergestellt oder kommerziell erhalten werden. Beispielhafte Capture-Antikörper sind die Anti-PrP-Antikörper SAF32 und SAF61 (Fa. Spi-Bio, Montigny le Bretonneux, Frankreich).

Als feste Phase können grundsätzlich alle festen Materialien dienen, die die Fixierung des Capture-Antikörpers ermöglichen, und die der Detektion der pathologischen Form des Prionproteins nicht im Wege stehen. Bevorzugt werden als feste Phasen Mikrotiterplatten oder magnetische oder nicht-magnetische Beads verwendet. Die Verwendung einer Mikrotiterplatte als feste Phase ist besonders bevorzugt.

Die Fixierung des Capture-Antikörpers an die feste Phase kann auf irgendeine, dem Fachmann bekannte Weise erfolgen. Der Capture-Antikörper kann dabei direkt an die feste Phase gebunden werden. Zum Beispiel kann der Capture-Antikörper kovalent an die feste Phase gekoppelt werden. Andererseits kann der Capture-Antikörper an der Oberfläche der festen Phase auch adsorbiert werden. Dazu wird er beispielsweise auf den Boden einer Kavität einer Mikrotiterplatte pipettiert und für einen geeigneten Zeitraum (zum Beispiel wenigstens 16 Stunden) bei einer geeigneten Temperatur (zum Beispiel 4°C) inkubiert. Es ist auch möglich, den Capture-Antikörper mit der festen Phase über das dem Fachmann bekannte Biotin/Avidin oder Streptavidin-System zu koppeln. Alternativ kann die Fixierung des Capture-Antikörpers auch über einen verbrückenden Antikörper erfolgen, der die Bindung des Capture-Antikörpers an die feste Phase vermittelt. Bevorzugt ist allerdings, dass der Capture-Antikörper direkt an der festen Phase fixiert wird.

Nach der Fixierung können freie Bindungsstellen der festen Phase mit einem geeigneten Blocking-Puffer gesättigt werden, dessen einzelne Bestandteile dem Fachmann bekannt sind. Ein geeigneter Blocking-Puffer kann beispielsweise aus einem geeigneten Puffersystem und einem Blocking-Reagenz, wie zum Beispiel Rinderserumalbumin (BSA) bestehen.

Nach der Fixierung der Capture-Antikörper an die feste Phase erfolgt die Inkubation mit der Probe. Dabei kommt es zur Bindung der pathologischen (PrP^{Sc}) und der nicht-pathologischen Form (PrP^{C}) des Prionproteins an die fixierten Capture-Antikörper. Die Inkubation erfolgt für einen Zeitraum, der ausreicht, damit beide Formen des Prionproteins möglichst quantitativ von den Capture-Antikörpern gebunden werden. Vorzugsweise beträgt die Inkubationszeit nicht mehr als 2 Stunden.

Nach der Inkubation wird die restliche Probe von den entstandenen Komplexen aus fester Phase, Capture-Antikörpern und Prionproteinen abgetrennt. Wird als feste Phase eine Mikrotiterplatte verwendet, dann kann die Entfernung der Probe zum Beispiel durch Absaugen erfolgen. Werden Beads als feste Phase verwendet, dann können die die Beads enthaltenden Komplexe durch Zentrifugation oder, im Fall von magnetischen Beads, unter Einwirkung von magnetischer Kraft sedimentiert, und die im Überstand befindliche Probe abgenommen werden.

Danach werden die Komplexe aus fester Phase, Capture-Antikörper und beiden Formen des Prionproteins mit Plasmin versetzt. Diesem Schritt liegt das entscheidende und überraschende Prinzip zugrunde, dass Plasmin die in den Komplexen enthaltene nicht-pathologische Form des Prionproteins (PrP^{Cµ}) spezifisch spaltet, die ebenfalls in den Komplexen enthaltene pathologische Form des Prionproteins (PrP^{Sc}) dagegen nicht. Nach der Spaltung der nichtpathologischen Form des Prionproteins enthalten die Komplexe die feste Phase, die Capture-Antikörper und die intakte, unverdaute pathologische Form des Prionproteins (PrP^{Sc}) bzw. das von den Capture-Antikörpern gebundene Spaltfragment der nicht-pathologischen Form des Prionproteins. Diejenigen Spaltfragmente der nicht-pathologischen Form des Prionproteins, die nicht von den Capture-Antikörpern gebunden werden, werden in diesem Schritt von den Komplexen entfernt.

Das für die Spaltung von PrP^{C} verwendete Plasmin ist nicht weiter eingeschränkt, mit der Ausnahme dass es in der Lage sein muss, die nicht-pathologische Form, nicht aber die pathologische Form des Prionproteins, spezifisch zu spalten. Es kann daher zum Beispiel rekombinantes oder natives Plasmin sein. Es kann auf dem Fachmann bekannte Weise, zum Beispiel durch Aktivierung von Plasminogen an einem Aktivator (zum Beispiel Urokinase oder Streptokinase), der zum Beispiel matrixgebunden sein kann, hergestellt sein. Es kann Human-Plasmin sein oder Plasmin aus anderen Spezies. Dem Fachmann ist klar, dass es möglich ist, in die Aminosäuresequenz von Plasmin auch Mutationen oder Deletionen einzuführen, ohne das dadurch die erfindungsgemäße Aktivität von Plasmin beeinträchtigt wird. Auch derart modifiziertes Plasmin wird von der vorliegenden Erfindung umfasst.

Für die Spaltung der nicht-pathologischen Form des Prionproteins liegt Plasmin bevorzugt in einer Lösung vor, die einen physiologischen Puffer, wie zum Beispiel PBS enthält. Die Konzentration von Plasmin wird so gewählt, dass sie für die Spaltung der nicht-pathologischen Form des in den Komplexen enthaltenen Prionproteins für das für die Spaltung vorgesehene Zeitintervall ausreichend ist. Die Konzentration von Plasmin beträgt vorzugsweise 10 nM bis 2 µM, noch bevorzugter 25 nM bis 1 µM, und noch mehr bevorzugt 40 nM bis 60 nM. Die Inkubationszeit der Komplexe mit Plasmin ist nicht besonders eingeschränkt. Bevorzugt beträgt diese Inkubationszeit allerdings nicht mehr als 30 Minuten.

In einer bevorzugten Ausführungsform wird die Spaltung der nichtpathologischen Form des Prionproteins durch die Zugabe eines geeigneten Reagenzes, das die Aktivität von Plasmin inhibiert, gestoppt. Bevorzugt wird hierfür Aprotinin verwendet. Das die Aktivität von Plasmin inhibierende Reagenz wird in fester oder bevorzugt flüssiger Form und in einer für die Inhibierung der Plasmin-Aktivität ausreichenden Konzentration zugegeben. Im Fall von Aprotinin beträgt die bevorzugte Konzentration 4 bis 6 µM.

Anschließend werden die durch die Inkubation mit Plasmin erhaltenen Spaltfragmente der nicht-pathologischen Form des Prionproteins, die nicht von dem Capture-Antikörper gebunden werden, von den Komplexen aus fester Phase, Capture-Antikörpern und der pathologischen Form des Prionproteins (PrP^{Sc}) bzw. dem durch die Spaltung mit Plasmin generierten und von den Capture-Antikörpern gebundenen Spaltfragments der nicht-pathologischen Form des Prionproteins (PrP^{C}) abgetrennt. Die Art und Weise der Abtrennung wird dabei an das verwendete Nachweissystem, vor allem an die verwendete feste Phase angepasst. In vielen Fällen ist es bevorzugt, die ungebundenen PrP^{C}-Spaltfragmente einfach durch Absaugen zu entfernen.

Nach der Abtrennung der ungebundenen PrP^{C}-Spaltfragmente von den Komplexen, wird das in den Komplexen enthaltene, nicht gespaltene Prionprotein mit Detektions-Antikörpern nachgewiesen. Bei dem nicht gespaltenen Prionprotein handelt es sich im Wesentlichen ausschließlich um die pathologische Form des Prionproteins (PrP^{Sc}). Die zu dessen Nachweis einzusetzenden Detektions-Antikörper weisen die Fähigkeit auf, Prp^{Sc} spezifisch zu binden. Da die nicht-pathologische Form des Prionproteins von den Komplexen im Wesentlichen vollständig entfernt wurde, kann als Detektions-Antikörper auch ein Antikörper verwendet werden, der sowohl Prp^{Sc} als auch PrP^{C} erkennt.

Die Detektion des Detektions-Antikörpers erfolgt auf irgendeine dem Fachmann bekannte Art und Weise. Hierzu ist aus dem Stand der Technik eine Vielzahl an geeigneten Detektionsverfahren bekannt. Als eine nicht erschöpfende Auswahl dieser Techniken seien ELISA (enzyme-linked immunosorbent assay), EIA (enzyme-linked immunoassay), Nanobeadstechnologie (zum Beispiel mit Europium markierte Nanobeads), Fluoreszenz- (zum Beispiel zeitaufgelöste Fluoreszenz) und Lumineszenzmethoden genannt.

Bevorzugt erfolgt die Detektion über dem Fachmann bekannte ELISA (enzyme-linked immunoabsorbent assay) -Techniken. Beispielsweise kann der Detektions-Antikörper mit Biotin konjugiert sein und dessen Detektion über ein Streptavidin-Polyperoxidase-Konjugat erfolgen, das unmittelbar vor der Messung mit Aktivatoren wie zum Beispiel Luminol oder TMB (3, 3', 5, 5'-Tetramethylbenzidin) versetzt wird. Bevorzugt erfolgt die Detektion über das Biotin/Streptavidin oder Avidin-System dann, wenn nicht bereits der Capture-Antikörper mit diesem System an die feste Phase fixiert worden ist. Beispiele für erfindungsgemäße Detektions-Antikörper sind die biotinylierten Anti-PrP-Antikörper SAF32 und SAF61 (Fa. Spi-Bio, Montigny le Bretonneux, Frankreich).

Dem Fachmann ist daher klar, das die Detektions-Antikörper mit einem Detektionsmolekül, einer für die Detektion geeigneten Gruppe oder auch mit festen Strukturen (zum Beispiel Mikrobeads oder Nanobeads, wie beispielsweise Europium-Nanobeads) konjugiert sein können, um den Nachweis mit einem der oben genannten oder weiteren aus dem Stand der Technik bekannten Nachweisverfahren zu ermöglichen. Es kann daher zum Beispiel bevorzugt sein, Detektions-Antikörper einzusetzen, die mit Biotin oder Fluoreszenzmarkern (wie zum Beispiel Fluorescein-Isothiocyanat oder Rhodamin) konjugiert sind. Die Detektions-Antikörper können zum Beispiel polyklonal oder bevorzugt monoklonal sein. Geeignete Detektions-Antikörper können nach Standardmethoden selbst hergestellt oder kommerziell erhalten werden.

In einer bevorzugten Ausführungsform werden der Capture-Antikörper und der Detektionsantikörper so gewählt, dass der Capture-Antikörper gegen ein Epitop des Prionproteins gerichtet ist, das aminoterminal zur Primärspaltstelle von Plasmin liegt, wenn der Detektionsantikörper ein Epitop des Prionproteins erkennt, das carboxyterminal zur Primärspaltstelle von Plasmin- angeordnet ist. Dementsprechend ist es auch bevorzugt, dass der Capture-Antikörper gegen ein Epitop des Prionproteins gerichtet ist, das carboxyterminal zur Primärspaltstelle von Plasmin liegt, wenn der Detektionsantikörper ein Epitop des Prionproteins erkennt, das aminoterminal zur Primärspaltstelle von Plasmin angeordnet ist.

In einer bevorzugten Ausführungsform werden somit Detektions-Antikörper und Capture-Antikörper so gewählt, dass der Capture-Antikörper gegen ein Epitop des Prionproteins gerichtet ist, das sich im Bereich der Aminosäurereste 1-110 befindet, wenn der Detektionsantikörper gegen ein Epitop gerichtet ist, das außerhalb dieses Bereichs liegt, oder der Detektionsantikörper gegen ein Epitop gerichtet ist, das sich im Bereich der Aminosäurereste 1-110 befindet, wenn der Capture-Antikörper gegen ein Epitop gerichtet ist, das außerhalb dieses Bereichs liegt.

In einer weiteren bevorzugten Ausführungsform erfolgt die Detektion des in den Komplexen enthaltenen, nicht gespaltenen Prionproteins quantitativ. Dies ist zum Beispiel möglich, wenn zur Detektion des Prionproteins ein ELISA-Test eingesetzt wird, bei dem die gemessene Signalintensität proportional zu der Menge des detektierten Prionproteins in der Probe ist. Wendet man das erfindungsgemäße Verfahren beispielsweise an Duplikaten von Proben an, und inkubiert die eine Probe mit einer für die vollständige Spaltung der nicht-pathologischen Form des Prionproteins ausreichenden Menge Plasmin für eine dafür ausreichende Zeit (zum Beispiel für 30 Minuten mit 50 nM Plasmin) und lässt die andere Probe unbehandelt, dann lässt sich anhand des Verhältnisses der Signalintensität der behandelten Probe zu der Signalintensität der unbehandelten Probe feststellen, zu welchem Ausmaß die Spaltung der Gesamtpopulation des Prionproteins erfolgt ist.

Für das erfindungsgemäße Verfahren kann es außerdem von Vorteil sein, wenn zwischen einem oder mehreren Einzelschritten des Verfahrens ein oder mehrere Waschschritte durchgeführt werden, für die dem Fachmann bekannte, geeignete Waschpuffer eingesetzt werden. Bevorzugt werden hierfür physiologische Pufferlösungen, wie PBS oder TBS eingesetzt, die mit Detergenzien wie Tween-20 supplementiert sein können.

Erfindungsgemäß können für die Durchführung des erfindungsgemäßen Verfahrens zum Nachweis von pathologischen Prionproteinen auch Kits eingesetzt werden.

Die erfindungsgemäßen Kits enthalten Capture-Antikörper, die gegen sowohl die pathologische (PrP^{Sc}) als auch die nicht-pathologische Form (PrP^{C}) des Prionproteins gerichtet sind, Plasmin und Detektions-Antikörper.

Die erfindungswesentlichen Merkmale dieser Bestandteile des Kits wurden bereits ausführlich beschrieben.

So kann es zum Beispiel vorteilhaft sein, wenn die Capture-Antikörper bereits auf einer festen Phase fixiert vorliegen. Als feste Phase dienen hierbei vorzugsweise Mikrotiterplatten oder magnetische oder nichtmagnetische Beads.

In einer Ausführungsform erkennen die im Kit enthaltenen Detektionsantikörper sowohl die pathologische als auch die nicht-pathologische Form des Prionproteins.

In einer bevorzugten Ausführungsform ist der im Kit enthaltene Capture-Antikörper gegen ein Epitop des Prionproteins gerichtet, das aminoterminal zur Primärspaltstelle von Plasmin liegt, wenn der Detektionsantikörper ein Epitop des Prionproteins erkennt, das carboxyterminal zur Primärspaltstelle von Plasmin angeordnet ist. Alternativ kann es auch bevorzugt sein, dass der Capture-Antikörper gegen ein Epitop des Prionproteins gerichtet ist, das carboxyterminal zur Primärspaltstelle von Plasmin liegt, wenn der Detektionsantikörper ein Epitop des Prionproteins erkennt, das aminoterminal zur Primärspaltstelle von Plasmin angeordnet ist.

In einer weiteren bevorzugten Ausführungsform ist der im Kit enthaltene Capture-Antikörper gegen ein Epitop des Prionproteins gerichtet ist, das sich im Bereich der Aminosäurereste 1-110 befindet, wenn der Detektionsantikörper gegen ein Epitop gerichtet ist, das außerhalb dieses Bereichs liegt. Andererseits kann der im Kit enthaltene Detektionsantikörper auch gegen ein Epitop gerichtet sein, das sich im Bereich der Aminosäurereste 1-110 befindet, wenn der Capture-Antikörper gegen ein Epitop gerichtet ist, das außerhalb dieses Bereichs liegt.

In einer noch weiteren bevorzugten Ausführungsform enthält der Kit zusätzlich einen Blocking-Puffer zur Sättigung freier Bindungsstellen der festen Phase, einen Waschpuffer und/oder Aprotinin.

In einer noch weiteren Ausführungsform liegt Plasmin in dem Kit entweder in einer Pufferlösung gelöst oder lyophilisiert als Feststoff vor.

Falls Aprotinin in dem Kit enthalten ist, kann dies ebenfalls in einer Pufferlösung gelöst oder lyophilisiert als Feststoff vorliegen. Eventuelle Zusätze zu den im Kit enthaltenen Lösungen (zum Beispiel Detergenzien, Blocking-Reagenzien) können ebenfalls im Kit enthalten sein.

Das erfindungsgemäße Verfahren erlaubt es, pathologische Prionproteine in einem frühen Krankheitsstadium bei geringem Kosten- und Zeitaufwand mit hoher Spezifität und Sensitivität gegebenenfalls im Rahmen eines automatisierten Tests nachzuweisen. Dabei gelingt der Nachweis von PrP^{Sc} in unterschiedlichen Spezies, wie Mensch, Hamster oder Rind mit außerordentlich hoher Sensitivität.

Durch die Bestimmung der ID₅₀-Dosis (die infektiöse Dosis, die in mindestens 50% der exponierten Tiere eine Erkrankung verursacht; Prusiner S., Proc. Natl. Acad. Sci. USA, 1998, 10, 95, 13363-13383) lässt sich die Sensitivität von Tests zum Nachweis von pathologischen Prionen ermitteln (je niedriger die ID₅₀ml-Wert, desto höher die Sensitivität des Tests). Mit dem erfindungsgemäßen Verfahren können Werte von weniger als 1.000 ID₅₀/ml erreicht werden. Zu Vergleichszwecken seien die ID₅₀/ml-Werte der kommerziell erhältlichen Tests Prionics Check (ID₅₀/ml: 1.000.000-100.000; BSE-Homogenat als Probe; Nachweisgrenze 10⁰-10⁻¹-Verdünnung), Platelia^{®} BSE-Test (ID₅₀/ml: 3.000; BSE-Homogenat als Probe; Nachweisgrenze 10^{-2,5}-Verdünnung) und Enfer TSA (ID₅₀/ml: 30.000; BSE-Homogenat als Probe; Nachweisgrenze 10^{-1,5}-Verdünnung) angegeben.

Das erfindungsgemäße Verfahren basiert auf der guten Spaltbarkeit der physiologischen PrP-Form durch Plasmin im Vergleich zu der schlechten Spaltbarkeit der pathologischen Form. Die spezifische Faltung des PrP^{Sc}-Moleküls, die die Primärspaltstelle für Plasmin verdeckt, und die höhere enzymatische Selektivität des Plasmins erlauben eine Unterscheidung zwischen den beiden Formen nach der Immobilisierung der Prionen. Die Verwendung von Plasmin hat den Vorteil gegenüber Proteinase K, dass nur PrP^{C} gespalten und nicht vollständig verdaut wird, wobei die verwendeten Antikörper intakt bleiben.

Das Verfahren dauert insgesamt etwa 3,5 Stunden und erfordert keine spezielle Vorbehandlung der Probe, wie z.B. der Platelia^{®} BSE-Test. Im Unterschied zum Enfer-Test, wo die Adsorption der Prionen unspezifisch auf der Oberfläche der Mikrotiterplatte erfolgt, ist die Bindung der Prionen von Anfang an spezifisch. Der Zeitaufwand beim erfindungsgemäßen Verfahren ist ferner deutlich geringer als beim Prionics-Check, bei dem der Nachweis nach einem Westem-Blot erfolgt.

Das erfindungsgemäße Verfahren vermindert die Abhängigkeit von einem spezifischen Antikörpertyp und ist somit äußerst flexibel: Da PrP^{C} grundsätzlich durch Plasmin in zwei Fragmente gespalten wird, können unterschiedliche Antikörper verwendet werden, so dass entweder die aminoterminale Region der die carboxyterminale Region des PrP-Moleküls nachgewiesen werden kann.

Ferner erlaubt das erfindungsgemäße Verfahren im Gegensatz zu allen anderen bisher bekannten Verfahren die Messung der Arifangsgeschwindigkeit der PrP^{C}-Spaltung. Das erfindungsgemäße Verfahren kann leicht automatisiert durchgeführt werden, was den Einsatz in der Routine begünstigt. Das Verfahren könnte zudem auch zur Erhöhung der Sensitivität anderer immunologischen Methoden zum Nachweis von Prion eingesetzt werden ("milde" Verdauung, dadurch besseres Signal-Rauschen-Verhältnis).

Die vorliegende Erfindung wird im Folgenden anhand von Beispielen illustriert, wobei diese jedoch nicht als einschränkend verstanden werden sollen.

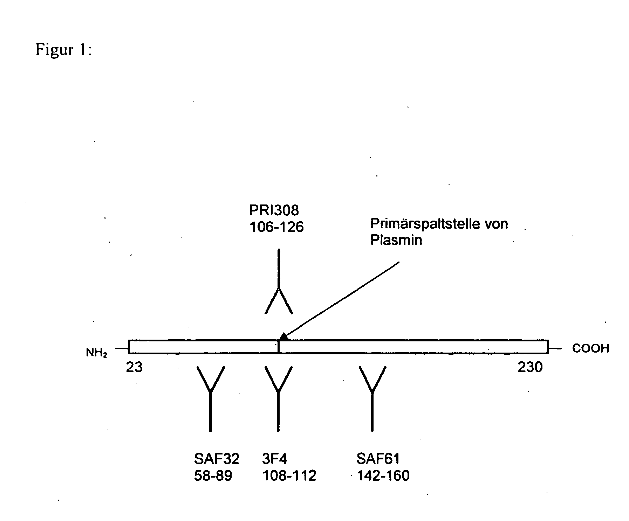

Figur 1 zeigt die Epitope der für die beispielhaften Versuche eingesetzten Anti-PrP-Antikörper.

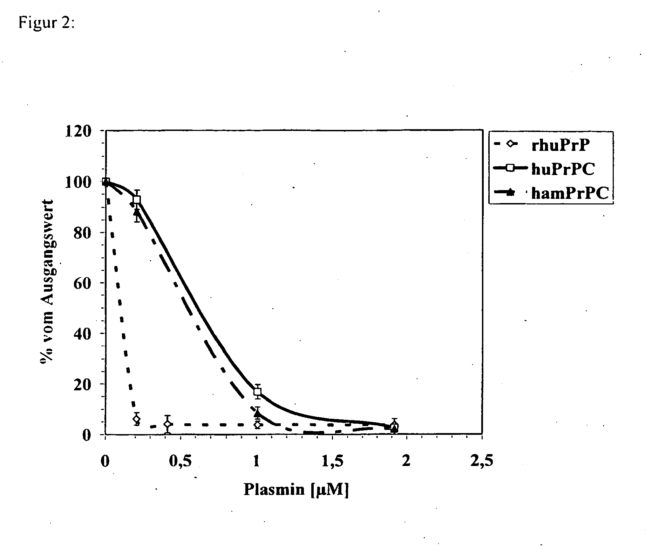

Figur 2 zeigt die in vitro-Spaltung von nicht immobilisierten Prionproteinen durch humanes Plasmin in Abhängigkeit von verschiedenen Plasmin-Konzentrationen. In der Legende bedeuten rhuPrP: rekombinantes humanes PrP, huPrP^{C}: humanes PrP^{C} (Serum), hamPrP^{C}: Hamster-PrP^{C} (Himhomogenisat). Es sind Mittelwerte und Standardabweichungen von drei unabhängigen Versuchen dargestellt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert.

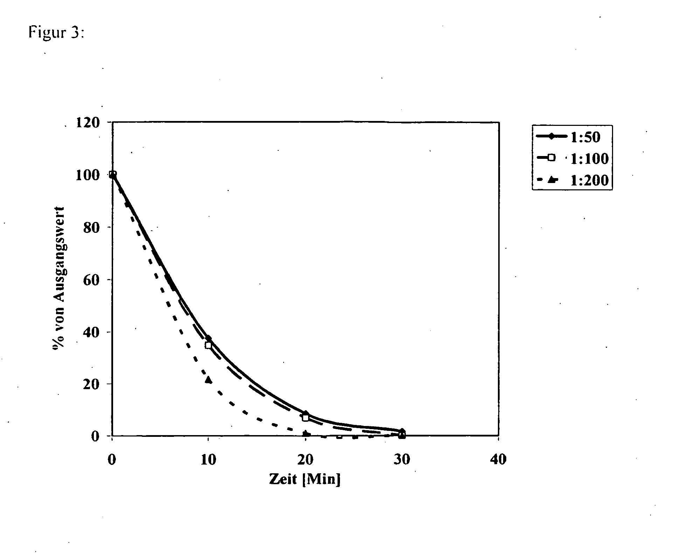

Figur 3 zeigt die Spaltung von nativem Human-PrP^{C} durch humanes Plasmin nach Immobilisierung auf der Mikrotiterplatte. Als Capture-Antikörper wurde SAF32 (erkennt Epitop zwischen den Aminosäureresten 58 und 89 des PrP-Moleküls) und als Detektionsantikörper biotinylierter 3F4 (erkennt Epitop zwischen den Aminosäureresten 108 und 111 des PrP-Moleküls) eingesetzt. Es wurden verschiedene Probenverdünnungen direkt auf der Platte mit Plasmin bei unterschiedlicher Inkubationsdauer bei 37°C behandelt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert.

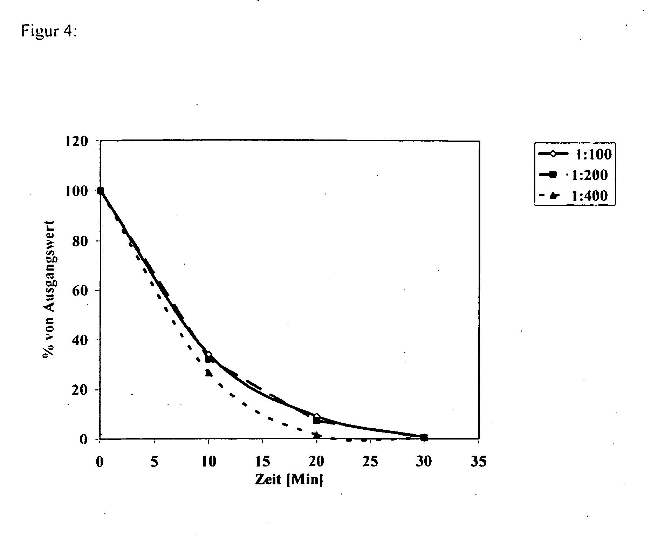

Figur 4 zeigt die Spaltung von nativem Hamster-PrP^{C} durch humanes Plasmin nach Immobilisierung auf der Mikrotiterplatte. Als Capture-Antikörper wurde SAF32 (erkennt Epitop zwischen den Aminosäureresten 58 und 89 des PrP-Moleküls) und als Detektionsantikörper biotinylierter 3F4 (erkennt Epitop zwischen den Aminosäureresten 108 und 111 des PrP-Moleküls) eingesetzt. Es wurden verschiedene Probenverdünnungen direkt auf der Platte mit Plasmin bei unterschiedlicher Inkubationsdauer und bei 37°C behandelt. Gezeigt ist das prozentuale Verhältnis der Intensität einer Probe nach der angegebenen Inkubationszeit mit Plasmin zur Intensität der Probe ohne Inkubation mit Plasmin.

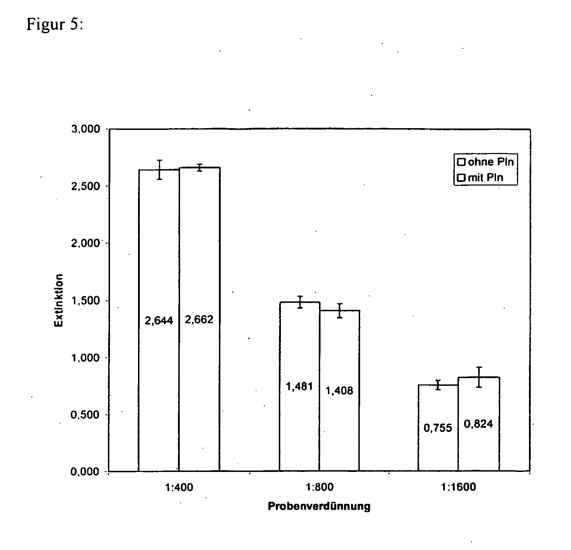

Figur 5 zeigt die Spaltung von nativem PrP^{C} (aus Hamster-Hirnhomogenisat) durch humanes Plasmin nach Immobilisierung auf einer Mikrotiterplatte. Als Capture-Antikörper wurde PRI308 (erkennt Epitop zwischen den Aminosäureresten 106-126 des PrP-Moleküls) und als Detektions-Antikörper biotinylierter SAF32 (erkennt Epitop zwischen den Aminosäureresten 58-89 des PrP-Moleküls) eingesetzt. Das PRI3O8-Epitop beinhaltet die Spaltstelle des Plasmins, wodurch die Spaltung von PrP^{C} unterdrückt wird.

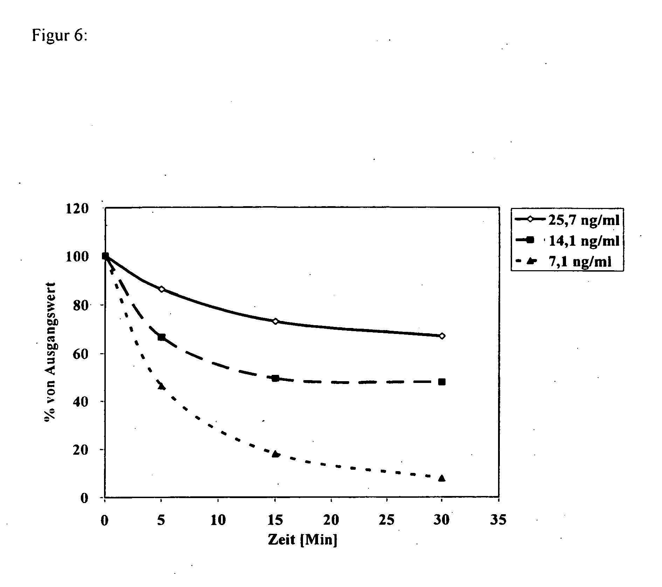

Figur 6 zeigt die Spaltung von rekombinantem Human-PrP mit ausgetauschten Lysinresten im Lysincluster 2 (dLC2) durch humanes Plasmin nach der Immobilisierung auf einer Mikrotiterplatte. Das Lysincluster 2 umfasst die Aminosäurereste 101 bis 110 des PrP. Die darin enthaltenen Lysinreste an Position 101, 104, 106 und 110 waren durch Alanin ausgetauscht. Es wurden unterschiedliche Probenkonzentrationen untersucht. Als Capture-Antikörper wurde SAF61 (erkennt Epitop zwischen den Aminosäureresten 142-160 des PrP-Moleküls) und als Detektionsantikörper biotinylierter SAF32 (erkennt Epitop zwischen den Aminosäureresten 58-89 des PrP-Moleküls) eingesetzt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert.

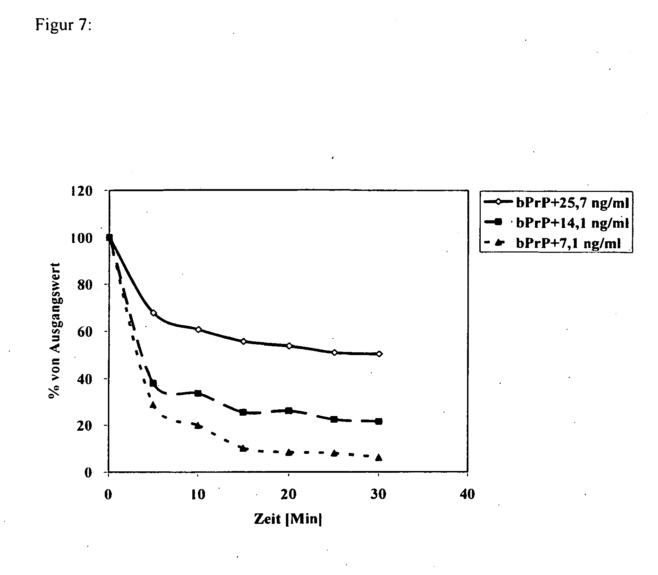

Figur 7 zeigt die Spaltung von rekombinantem Human-PrP mit ausgetauschten Lysinresten im Lysincluster 2 (dLC2) durch humanes Plasmin nach der Immobilisierung auf einer Mikrotiterplatte im Überschuß von bPrP (bovines PrP aus Rinderhirnhomogenisat). Als Capture-Antikörper wurde SAF61 (erkennt Epitop zwischen den Aminosäureresten 142-160 des PrP-Moleküls) und als Detektionsantikörper biotinylierter SAF32 (erkennt Epitop zwischen den Aminosäureresten 58-89 des PrP-Moleküls) eingesetzt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert.

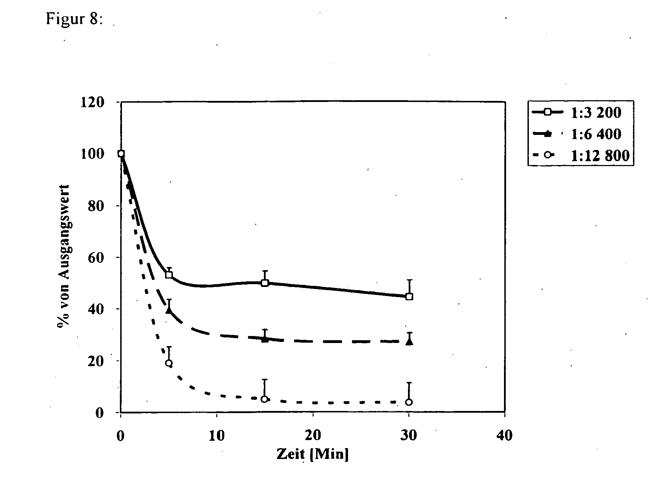

Figur 8 zeigt die Spaltung von PrP^{Sc} aus Hamster-Hirnhomogenisat durch humanes Plasmin nach der Immobilisierung auf einer Mikrotiterplatte. Als Capture-Antikörper wurde SAF61 (erkennt Epitop zwischen den Aminosäureresten 142-160 des PrP-Moleküls) und als Detektionsantikörper SAF32-Biotin (erkennt Epitop zwischen Aminosäureresten 58-89 des PrP-Moleküls) eingesetzt. Es sind verschiedene Verdünnungen von Hirnhomogenisaten von mit Scrapie infizierten Tieren dargestellt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert. Es sind Mittelwerte und Standardabweichungen von drei unabhängigen Versuchen dargestellt.

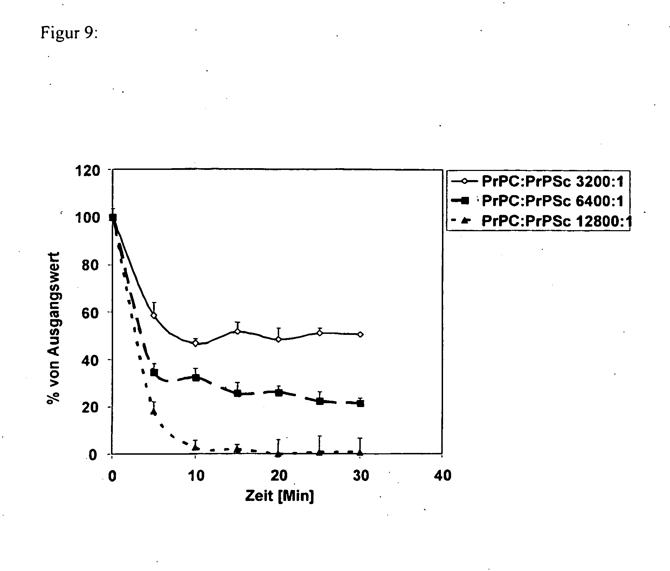

Figur 9: Spaltung von PrP^{Sc} (aus Hamster-Hirnhomogenisat) im Überschuss von nativem PrP^{C} durch humanes Plasmin nach Immobilisierung auf einer Mikrotiterplatte. Als Capture-Antikörper wurde SAF61 (erkennt Epitop zwischen den Aminosäureresten 142-160 des PrP-Moleküls) und als Detektionsantikörper biotinylierter SAF32 (erkennt Epitop zwischen den Aminosäureresten 58-89 des PrP-Moleküls) eingesetzt. Es bedeuten PrP^{C} die normale, zelluläre Form von PrP und PrP^{Sc} die pathologische Form von PrP. Das Hirnhomogenisat aus Scrapie-Hamster wurde in Hirnhomogenisat aus gesunden Hamstern verdünnt. Es sind Mittelwerte und Standardabweichungen von drei unabhängigen Versuchen dargestellt. Gezeigt ist das prozentuale Verhältnis der Intensität einer Probe nach der angegebenen Inkubationszeit mit Plasmin zur Intensität der Probe ohne Inkubation mit Plasmin. Sämtliche angegebenen Werte sind hintergrundkorrigiert

### Beispiel 1: Spaltung von nicht immobilisiertem PrP durch Plasmin

In einer Reihe von Versuchen wurde sowohl das rekombinante humane PrP (rhuPrP), als auch natives PrP^{C} (Humanserum, Hamster-Hirnhomogenisat) in vitro mit Plasmin gespalten, wobei der Plasmin-Verdau allerdings in einem Proberöhrchen erfolgte, und die Prionproteine zum Zeitpunkt des Verdaus nicht immobilisiert waren.

Rekombinantes humanes PrP (rhuPrP), humanes PrP^{C} (Serum; huPrP^{C}) und Hamster PrP^{C} (Hirnhomogenisat; hamPrP^{C}) wurden in Ausgangskonzentrationen von 553 bis 575 pg/ml (Kalibrierung gegen rekombinantes humanes PrP; Fa. Roboscreen) für 30 Minuten bei 37°C mit unterschiedlichen Konzentrationen an humanem Plasmin in einem Proberöhrchen inkubiert. Anschließend wurde die Reaktion durch Zugabe von Aprotinin gestoppt und PrP durch einen ELISA-Test nachgewiesen. Als Capture-Antikörper wurde SAF32 (erkennt Epitop zwischen den Aminosäureresten 58 und 89 des PrP-Moleküls) und als Detektionsantikörper mit Biotin gekoppelter 3F4 (erkennt Epitop zwischen den Aminosäureresten 108 und 111 des PrP-Moleküls) eingesetzt. Für den ELISA-Test wurde die Probe mit einem Streptavidin-Polyperoxidase-Konjugat (SApolyHRP, Fa. Pierce, Rockford, USA), das in Reaktionspuffer (1 Teil Blocking-Puffer + 4 Teile PBS) 1:5000 verdünnt war, versetzt, und für 20 Minuten bei Raumtemperatur inkubiert. Anschließend wurde TMB (3, 3', 5, 5'-Tetramethylbenzidin) als Substrat zu der Probe gegeben und für 30 Minuten inkubiert. Nach der Zugabe von Stopplösung (0,25% H₂SO₄ in destilliertem Wasser) wurde die Extinktion der Probe bei 405 nm mit einem ELISA-Reader (Tecan Genios; Tecan, Schweiz) gemessen.

Das Epitop des Detektionsantikörpers liegt genau in der Primärspaltstelle des Prionproteins, wodurch lediglich nicht gespaltene Prionproteine in der Probe durch ELISA detektiert werden können (Fig. 1).

Es zeigte sich, dass die Spaltung von nativem PrP^{C} in vitro durch die Gegenwart von in der Probe vorhandenen Plasmin-Inhibitoren nur schwer zu kontrollieren ist, wenn der Verdau in einem Proberöhrchen ohne vorherige Immobilisierung der Prionproteine durchgeführt wird. Für eine signifikante Spaltung von rekombinantem humanem PrP im Teströhrchen ist demnach eine Plasmin-Konzentration von etwa 200 nM nötig, während das gleiche Maß an Spaltung für Human-PrP^{C} und Hamster-PrP^{C} erst bei einer Plasmin-Konzentration von über 1 µM erreicht wird (Fig. 2).

Aus diesem Grund wurde ein Test entwickelt, bei dem in einer Probe enthaltene Prionen zunächst mittels eines monoklonalen Antikörpers immobilisiert, dann mit humanem Plasmin behandelt und die restlichen, nicht gespaltenen Prionen anschließend mit einem anderen markierten Antikörper nachgewiesen werden. Da die Probe vor dem Plasmin-Verdau von den Komplexen aus fixierten Antikörpern und Prionproteinen getrennt wird, können in der Probe enthaltene Plasmin-Inhibitoren bzw. Plasmin-Substrate die Plasmin-Aktivität beim Verdau nicht inhibieren oder beeinflussen.

### Beispiel 2: Spaltung vom immobilisiertem PrP durch Plasmin

In weiteren Versuchen wurde die Spaltung von PrP durch Plasmin nach Immobilisierung mittels auf Mikrotiterplatten fixierten Antikörpern untersucht (Fig. 3 und Fig. 4). Dafür wurden sowohl Antikörper verwendet, die gegen unterschiedliche Epitope des PrP-Moleküls gerichtet sind (Fig. 1; Fig. 5), als auch rhuPrP eingesetzt, bei dem die Lysin-Reste in der Spaltregion des Plasmins durch Alanin ausgetauscht sind (Fig. 6 und Fig. 7).

### Beispiel 2.1: Spaltung von immobilisiertem PrP^{C} aus Humanplasma bzw. Hamsterhirnhomogenat

Zur Spaltung von immobilisiertem PrP^{C} aus Humanplasma wurden drei transparente Mikrotiterplatten (Lumi-Nunc Maxi-Sorp F96; Fa. Nunc, Wiesbaden) mit 100 µl/Kavität monoklonalem Antikörper (Anti-PrP: SAF32, Fa. Spi-Bio, Montigny le Bretonneux, Frankreich; Konzentration: 1 µg/ml in Carbonat/Bicarbonat Puffer pH 9,0, Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio, Bonn) in jede Kavität für 1 Stunde saturiert. Der Blocking-Puffer wurde von den Platten abgesaugt und die Proben (gepooltes humanes Citratplasma, entsprechend verdünnt in Reaktionspuffer: 1 Teil Blocking-Puffer + 4 Teile PBS) auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. In die Kavitäten wurden anschließend 100 µl 50 nM humanes Plasmin (Fa. Chromogenix, Stockholm, Schweden) in PBS (Fa. Perbio, Bonn) pipettiert. Die Platten wurden für 0, 10, 20 und 30 Minuten bei 37°C in einem Thermoschüttler (THERMOSTAR, Fa. BMG, Offenburg) bei 500 Umdrehungen pro Minute inkubiert. Danach wurden in jede Kavität 25 µl 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion des unverdauten PrP wurden 100 µl biotinylierter Detektionsantikörper (3F4, Fa. Signet, Dedham, USA; 125 ng/ml) in Reaktionspuffer in jede Kavität pipettiert.

### ELISA-Test:

Für den ELISA-Test wurde mit dem Detektionsantikörper für 1 Stunde bei Raumtemperatur unter leichtem Schütteln im Dunkeln inkubiert. Nach sechsmaligem Waschen mit jeweils 300 µl Waschpuffer (TBS (Burph TBS, Fa. Pierce, Rockford, USA) mit 0,5% Tween-20 (Surfact-Amps, Fa. Pierce, Rockford, USA)) wurde ein Streptavidin-Polyperoxidase-Konjugat (SApolyHRP, Fa. Pierce, Rockford, USA) in Reaktionspuffer (1 Teil Blocking-Puffer + 4 Teile PBS) 1:5000 verdünnt und zu 100 µl pro Kavität auf die Mikrotiterplatten pipettiert. Nach 20 Minuten Inkubation bei Raumtemperatur unter leichtem Schütteln im Dunkeln wurde wiederum sechsmal mit jeweils 300 µl Waschpuffer gewaschen. Anschließend wurden jeweils 100 µl auf Raumtemperatur gebrachtes TMB (3, 3', 5, 5'-Tetramethylbenzidin) als Substrat in jede Kavität pipettiert und für 30 Minuten bei Raumtemperatur unter mäßigem Schütteln im Dunkeln inkubiert. Nach der Zugabe von 50 µl Stopplösung (0,25% H₂SO₄ in destilliertem Wasser) je Kavität wurde die Extinktion der Probe bei 405 nm mit einem ELISA-Reader (Tecan Genios; Tecan, Schweiz) gemessen.

Die Ergebnisse der Spaltung von immobilisiertem PrP^{C} aus Humanplasma sind in Fig. 3 dargestellt. Analog dazu wurde auch die Spaltung von immobilisiertem PrP^{C} aus Hamsterhirnhomogenat durchgeführt, mit der einzigen Ausnahme, dass für die Probe nicht gepooltes humanes Citratplasma, sondern Hamsterhirnhomogenat aus gesunden Tieren verwendet wurde. Die Ergebnisse der Spaltung von immobilisiertem PrP^{C} aus Hamsterhirnhomogenat sind in Fig. 4 gezeigt.

Die exponential abnehmende prozentuale Intensität, die in den Fig. 3 und 4 erkennbar ist, macht deutlich, dass der als Detektionsantikörper verwendete biotinylierte 3F4, der ein Epitop zwischen den Aminosäureresten 108 und 111 des PrP-Moleküls erkennt, mit fortschreitender Inkubationsdauer immer weniger Epitope detektiert. Dies zeigt, dass das Epitop dieses PrP-Antikörpers auf dem PrP^{C}-Fragment liegt, das durch die Spaltung mit Plasmin abgebaut und vor dem Nachweis mit dem Detektionsantikörper ausgewaschen wurde.

Es zeigte sich ferner, dass bereits nach zwanzig Minuten Inkubation mit Plasmin die Gesamtmenge des immobilisierten PrP^{C} aus Humanplasma der 1:200-Verdünnung durch Plasmin gespalten war. Bei der 1:50- und der 1:100-Verdünnung war nach 30 Minuten Plasmin-Inkubation praktisch kein ungespaltenes Human-PrP^{C} mehr vorhanden (Fig. 3).

Beim Hamsterhomogenat (1:400-Verdünnung) war nach 20 Minuten Plasmin-Inkubation die Gesamtmenge des immobilisierten PrP^{C} durch Plasmin gespalten. Bei der 1:100- und der 1:200-Verdünnung war nach 30 Minuten Inkubation mit Plasmin praktisch kein ungespaltenes Hamster-PrP^{C} mehr detektierbar (Fig. 4).

### Beispiel 2.2: Unterdrückung der Spaltung von PrP

Zur Unterdrückung der PrP-Spaltung durch Plasmin wurden zwei transparente Mikrotiterplatten (Lumi-Nunc Maxi-Sorp F96; Fa. Nunc, Wiesbaden) mit 100 µl/Kavität monoklonalem Antikörper (Anti-PrP: PRI308, Fa. Spi-Bio, Montigny le Bretonneux, Frankreich; Konzentration: 1 µg/ml in Carbonat/Bicarbonat Puffer pH 9,0 (Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio, Bonn) in jede Kavität für 1 Stunde saturiert. Der Blocking-Puffer wurde von den Platten abgesaugt und die Proben (Hamsterhinhomogenatextrakt aus gesunden Tieren, entsprechend verdünnt in Reaktionspuffer: 1 Teil Blocking-Puffer + 4 Teile PBS) auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. Auf eine Mikrotiterplatte wurden anschließend 100 µl 50 nM humanes Plasmin (Fa. Chromogenix, Stockholm, Schweden) in PBS (Fa. Perbio, Bonn) pipettiert, während auf die zweite Mikrotiterplatte 100 µl PBS pipettiert wurden. Die Platten wurden für 30 Minuten bei 37°C in einem Thermoschüttler (THERMOSTAR, Fa. BMG, Offenburg) bei 500 Umdrehungen pro Minute inkubiert. Danach wurden auf beide Platten 25 µl/Kavität 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion des unverdauten PrP wurden 100 µl biotinylierter Detektionsantikörper (SAF32, Fa. Spi-Bio, Montigny le Bretonneux, Frankreich; 125 ng/ml) in Reaktionspuffer in jede Kavität pipettiert. Im Anschluss wurde der ELISA-Test gemäß obiger Vorschrift durchgeführt.

Wie aus Fig. 5 ersichtlich ist, ist nach der Immobilisierung von nativem PrP^{C} mittels eines Capture-Antikörpers (PRI3O8), der als Epitop die Aminosäurereste 106-126 des PrP-Moleküls erkennt, keine Spaltung mit Plasmin möglich. Dies zeigt, dass durch die Bindung von PrP^{C} durch den Capture-Antikörper die Primärspaltstelle von PrP^{C} für Plasmin maskiert ist, und sich diese zwischen den Aminosäureresten 106-126 des PrP-Moleküls befinden muss.

### Beispiel 2.3: Spaltung von rekombinantem PrP^{C}

Zur Spaltung von rekombinantem PrP^{C} wurden vier transparente Mikrotiterplatten (Lumi-Nunc Maxi-Sorp F96; Fa. Nunc, Wiesbaden) mit 100 µl/Kavität monoklonalem Antikörper (Anti-PrP: SAF61, Fa. Spi-Bio, Montigny le Bretonneux, Frankreich; Konzentration 1 µg/ml in Carbonat/Bicarbonat Puffer pH 9,0, Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio, Bonn) in jede Kavität für 1 Stunde saturiert. Der Blocking-Puffer wurde von den Platten abgesaugt und die Proben (rekombinantes humanes PrP mit durch Alanin ausgetauschten Lysinresten im Lysin-Cluster 2 (dLC2), vom Institut für Labormedizin, Charité, Campus Virchow Klinikum; entsprechend verdünnt in Reaktionspuffer: 1 Teil Blocking-Puffer + 4 Teile PBS) wurden auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. In die Mikrotiterplatten wurden anschließend 100 µl 50 nM humanes Plasmin (Fa. Chromogenix, Stockholm, Schweden) in PBS (Fa. Perbio, Bonn) pipettiert. Die Platten wurden für 0, 5, 15 und 30 Minuten bei 37°C in einem Thermoschüttler (THERMOSTAR, Fa. BMG, Offenburg) bei 500 Umdrehungen pro Minute inkubiert. Danach wurden auf alle Platten 25 µl/Kavität 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion des unverdauten PrP^{C} wurden 100 µl biotinylierter Detektionsantikörper (SAF32, Fa. Spi-Bio, Montigny le Bretonneux, Frankreich; 125 ng/ml) in Reaktionspuffer in jede Kavität pipettiert. Im Anschluss wurde der ELISA-Test gemäß obiger Vorschrift durchgeführt.

Die Ergebnisse (Fig. 6) zeigen, dass der Austausch von Lysinresten im Lysin-Cluster 2 (dLC2) von humanem PrP durch Alanin zu einer signifikanten Beeinträchtigung der Spaltung von PrP durch Plasmin führt. So sind in der Probe, die das mutierte PrP^{C} in einer Konzentration von 25,7 ng/ml enthielt, nach 30 Minuten noch über 70% des mutierten PrP^{C} unverdaut. Auch bei geringeren Konzentrationen des mutierten PrP^{C} ist die Plasmin-Spaltung stark gehemmt.

### Beispiel 2.4: Spaltung von immobilisiertem rekombinantem und nativem PrP^{C} in einem Gemisch

Zur Spaltung von immobilisiertem rekombinantem und nativem PrP^{C} in einem Gemisch wurden sieben transparente Mikrotiterplatten (Lumi-Nunc Maxi-Sorp F96; Fa. Nunc, Wiesbaden) mit 100 µl/Kavität monoklonalem Antikörper (Anti-PrP: SAF61, Fa. Spi-Bio, Montigny le Bretonneux, Frankreich; Konzentration 1 µg/ml in Carbonat/Bicarbonat Puffer pH 9,0, Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio, Bonn) in jede Kavität für 1 Stunde saturiert. Der Blocking-Puffer wurde von den Platten abgesaugt und die Proben (rekombinantes humanes PrP mit durch Alanin ausgetauschten Lysinresten im Lysin-Cluster 2 (dLC2), vom Institut für Labormedizin, Charité, Campus Virchow Klinikum; verdünnt auf 25,7; 14,1 und 7,1 ng/ml in Rinderhimhomogenisat, 1:100 verdünnt in Reaktionspuffer; Reaktionspuffer: 1 Teil Blocking-Puffer + 4 Teile PBS) wurden auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. In die Mikrotiterplatten wurden anschließend 100 µl 50 nM humanes Plasmin (Fa. Chromogenix, Stockholm, Schweden) in PBS (Fa. Perbio, Bonn) pipettiert. Die Platten wurden für 0, 5, 10, 15, 20, 25 und 30 Minuten bei 37°C in einem Thermoschüttler (THERMOSTAR, Fa. BMG, Offenburg) bei 500 Umdrehungen pro Minute inkubiert. Danach wurden auf alle Platten 25 µl/Kavität 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion des unverdauten PrP^{C} wurden 100 µl biotinylierter Detektorantikörper (SAF32, Fa. Spi-Bio, Montigny le Bretonneux, Frankreich; 125 ng/ml) in Reaktionspuffer in jede Kavität pipettiert. Im Anschluss wurde der ELISA-Test gemäß obiger Vorschrift durchgeführt.

Aus Fig. 7 ist ersichtlich, dass natives Rinder-PrP^{C} auch aus einem Gemisch mit unterschiedlichen Konzentration an mutiertem, rekombinantem Human-PrP, dessen Spaltung durch Plasmin aufgrund der Mutation stark unterdrückt ist (siehe vorangegangenes Beispiel), gespalten werden kann.

### Beispiel 3: Test zum Nachweis von PrP^{Sc} in einer Probe mit PrP^{C}

In einer weiteren Reihe von Versuchen wurden Scrapie-Proben (Hamster-263K-Stamm) mit Plasmin untersucht. Es wurde Hirnhomogenisat aus infizierten Tieren im Terminalstadium in verschiedenen Konzentrationen auf die Gegenwart von PrP^{Sc} hin untersucht (Fig. 8). Außerdem wurden verschiedene Mengen von Hirnhomogenisat aus infizierten Tieren zu Hirnhomogenisat aus gesunden Tieren zugegeben, um den Nachweis der pathologischen Form in Anwesenheit von größeren Konzentrationen der normalen Form zu überprüfen (Fig. 9).

### Beispiel 3.1: Spaltung von PrP^{C} aus Hamsterhirnhomogenat von infizierten Tieren durch Plasmin nach Immobilisierung auf einer Mikrotiterplatte

Zur Vorbereitung der Proben wurden 40 µl Hamsterhirnhomogenat aus mit Scrapie infizierten Tieren mit 40 µl 2% Sarkosyl (Fa. Sigma, Seelze) gemischt, für 60 Sekunden bei Stufe 3 mit Ultraschall behandelt (Ultraschallgerät UP100H, Fa. Dr. Hielscher, Teltow) und für 2 Stunden bei Raumtemperatur auf einem Rotor (neoLab-Rotor, Fa. Roth, Karlsruhe) inkubiert. Danach wurde eine Verdünnungsreihe in Reaktionspuffer vorbereitet.

Vier transparente Mikrotiterplatten (Lumi-Nunc Maxi-Sorp F96; Fa. Nunc, Wiesbaden) wurden mit 100 µl/Kavität monoklonalem Antikörper (Anti-PrP: SAF61, Fa. Spi-Bio, Montigny le Bretonneux, Frankreich; Konzentration 1 µg/ml in Carbonat/Bicarbonat Puffer pH 9,0, Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio. Bonn) in jede Kavität für 1 Stunde saturiert. Der Blocking-Puffer wurde von den Platten abgesaugt und die vorbereiteten Proben wurden auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. In die Mikrotiterplatten wurden anschließend 100 µl 50 nM humanes Plasmin (Fa. Chromogenix, Stockholm, Schweden) in PBS (Fa. Perbio, Bonn) pipettiert. Die Platten wurden für 5, 15 und 30 Minuten bei 37°C in einem Thermoschüttler (THERMOSTAR, Fa. BMG, Offenburg) bei 500 Umdrehungen pro Minute inkubiert. Danach wurden auf alle Platten 25 µl/Kavität 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion unverdauter PrP-Moleküle wurden 100 µl biotinylierter Detektionsantikörper (SAF32, Fa. Spi-Bio, Montigny le Bretonneux, Frankreich; 125 ng/ml) in Reaktionspuffer in jede Kavität pipettiert. Im Anschluss wurde der ELISA-Test gemäß obiger Vorschrift durchgeführt.

Es zeigte sich, dass PrP^{Sc} selbst in einer 1:6400-Verdünnung des Hamsterhirnhomogenats von mit Scrapie infizierten Tieren in Puffer noch deutlich nachzuweisen ist. Die gemessene Intensität ist proportional zur Konzentration des Hamsterhirnhomogenats (und damit des PrP^{Sc}).

### Beispiel 3.2: Spalten von PrP^{Sc} aus Hamsterhirnhomogenat im Überschuss von PrP^{C} durch Plasmin nach Immobilisierung auf einer Mikrotiterplatte

Zur Vorbereitung der Proben wurden 40 µl Hamsterhirnhomogenat aus mit Scrapie infizierten Tieren bzw. aus gesunden Tieren mit 40 µl 2% Sarkosyl (Fa. Sigma, Seelze) gemischt, für 60 Sekunden bei Stufe 3 mit Ultraschall behandelt (Ultraschallgerät UP100H, Fa. Dr. Hielscher, Teltow) und für 2 Stunden bei Raumtemperatur auf einem Rotor (neoLab-Rotor Fa. Roth, Karlsruhe) inkubiert. Danach wurde eine Verdünnungsreihe von Scrapie-Homogenisaten in Normal-Homgenisaten vorbereitet. Unmittelbar vor dem Pipettieren auf die Platten wurden die Proben 1:100 in Reaktionspuffer verdünnt.

Sieben transparente Mikrotiterplatten (Lumi-Nunc Maxi-Sorp F96; Fa. Nunc, Wiesbaden) wurden mit 100 µl/Kavität monoklonalem Antikörper (Anti-PrP: SAF61, Fa. Spi-Bio, Montigny le Bretonneux, Frankreich; Konzentration 1 µg/ml in Carbonat/Bicarbonat Puffer pH 9,0, Fa. Perbio, Bonn) für 16-18 Stunden bei 4°C beschichtet. Die Restflüssigkeit wurde abgesaugt und die freien Bindungsstellen durch Zugabe von 100 µl Blocking-Puffer (Superblock, Fa. Perbio, Bonn) in jede Kavität für 1 Stunde saturiert. Der Blocking-Puffer wurde von den Platten abgesaugt und die vorbereiteten Proben wurden auf die Platten pipettiert. Nach zweistündiger Inkubation bei Raumtemperatur wurden die Platten dreimal mit Waschpuffer (TBS (Burph TBS, Fa. Pierce, Rockford, USA) mit 0,5% Tween-20 (Surfact-Amps, Fa. Pierce, Rockford, USA)) gewaschen. In die Mikrotiterplatten wurden anschließend 100 µl 50 nM humanes Plasmin (Fa. Chromogenix, Stockholm, Schweden) in PBS (Fa. Perbio, Bonn) pipettiert. Die Platten wurden für 5, 15 und 30 Minuten bei 37°C in einem Thermoschüttler (THERMOSTAR, Fa. BMG, Offenburg) bei 500 Umdrehungen pro Minute inkubiert. Danach wurden auf alle Platten 25 µl/Kavität 5 µM Aprotinin (Fa. Merck Biosciences, Schwalbach) in PBS pipettiert. Nach 5 Minuten Inkubation bei Raumtemperatur wurden die Platten dreimal mit dem Waschpuffer gewaschen. Zur Detektion unverdauter PrP-Moleküle wurden 100 µl biotinylierter Detektionsantikörper (SAF32, Fa. Spi-Bio, Montigny le Bretonneux, Frankreich; 125 ng/ml) in Reaktionspuffer in jede Kavität pipettiert. Im Anschluss wurde der ELISA-Test gemäß obiger Vorschrift durchgeführt.

Aus Fig. 9 ist ersichtlich, dass die pathologische Form des Prionproteins (PrP^{Sc}) selbst bei 6400-fachem Überschuss der nicht-pathologischen Form des Prionproteins (PrP^{C}) noch deutlich nachgewiesen werden kann. Dies zeigt, dass der Nachweis von PrP^{Sc} durch das erfindungsgemäße Verfahren nicht nur an Subjekten gelingt, die sich im terminalen Krankheitsstadium befinden, in dem die Konzentration an PrP^{Sc} die Konzentration von PrP^{C} übersteigt, sondern bereits deutlich vorher, in einem frühen vorklinischen Stadium möglich ist.

## Patentansprüche

1. Verfahren zum Nachweis von pathologischen Prionproteinen in vitro in einer Probe, wobei man:
a) Capture-Antikörper, die sowohl die pathologische (PrP^{Sc}) als auch die nicht-pathologische Form (PrP^{C}) des Prionproteins erkennen, auf einer festen Phase fixiert;
b) die Probe mit den fixierten Antikörpern aus Schritt a) inkubiert, wobei die pathologische und die nicht-pathologische Form des Prionproteins an die fixierten Antikörper unter Bildung von Komplexen binden;
c) die Probe von den entstandenen Komplexen abtrennt;
d) die Komplexe mit Plasmin inkubiert, wobei die nicht-pathologische Form des Prionproteins gespalten wird;
e) die durch die Inkubation mit Plasmin erhaltenen Spaltfragmente von den Komplexen abtrennt; und
f) das in den Komplexen enthaltene, nicht gespaltene Prionprotein mit Detektions-Antikörpern detektiert, welche die Fähigkeit aufweisen, PrP^{Sc} spezifisch zu binden.

2. Verfahren nach Anspruch 1, wobei die Detektion in Schritt f) quantitativ erfolgt.

3. Verfahren nach Anspruch 1, wobei die Detektions-Antikörper sowohl die pathologische als auch die nicht-pathologische Form des Prionproteins erkennen.

4. Verfahren nach Anspruch 1, wobei der Capture-Antikörper gegen ein Epitop des Prionproteins gerichtet ist, das sich im Bereich der Aminosäurereste 1-110 befindet, wenn der Detektionsantikörper gegen ein Epitop gerichtet ist, das außerhalb dieses Bereichs liegt, oder der Detektionsantikörper gegen ein Epitop gerichtet ist, das sich im Bereich der Aminosäurereste 1-110 befindet, wenn der Capture-Antikörper gegen ein Epitop gerichtet ist, das außerhalb dieses Bereichs liegt.

5. Verfahren nach einem der vorangegangenen Ansprüche, wobei nach Schritt a) freie Bindungsstellen der festen Phase durch Blocking-Puffer gesättigt werden.

6. Verfahren nach einem der vorangegangenen Ansprüche, wobei die festen Phasen Mikrotiterplatten oder paramagnetische oder nicht-magnetische Beads sind.

7. Verfahren nach einem der vorangegangenen Ansprüche, wobei die Inkubationszeit in Schritt b) nicht mehr als 2 Stunden beträgt.

8. Verfahren nach einem der vorangegangenen Ansprüche, wobei die Inkubationszeit in Schritt d) nicht mehr als 30 Minuten beträgt.

9. Verfahren nach einem der vorangegangenen Ansprüche, wobei auf die Inkubation mit Plasmin in Schritt d) die Zugabe von Aprotinin folgt.

10. Verfahren nach einem der vorangegangenen Ansprüche, wobei zwischen den einzelnen Schritten Waschschritte durchgeführt werden.

11. Verfahren nach einem der vorangegangenen Ansprüche, wobei die Detektionsantikörper Biotin, Fluoreszenzmarker und/oder Nanobeads, insbesondere mit Europium markierte Nanobeads, enthalten.

12. Verfahren nach einem der vorangegangenen Ansprüche, wobei die Detektion in Schritt f) durch einen ELISA-Test erfolgt.

13. Diagnostischer Kit zum Nachweis von pathologischen Prionen in vitro in einer Probe, enthaltend:
a) Capture-Antikörper, die gegen sowohl die pathologische (PrP^{Sc}) als auch die nicht-pathologische Form (PrP^{c}) des Prionproteins gerichtet sind,
b) Plasmin; und
c) Detektions-Antikörper, welche die Fähigkeit aufweisen, PrP^{Sc} spezifisch zu binden.

14. Diagnostischer Kit nach Anspruch 13, wobei die Capture-Antikörper bereits auf einer festen Phase fixiert sind.

15. Diagnostischer Kit nach Anspruch 14, wobei die feste Phase Mikrotiterplatten oder Beads sind.

16. Diagnostischer Kit nach Anspruch 13, wobei die Detektionsantikörper sowohl die pathologische als auch die nicht-pathologische Form des Prionproteins erkennen.

17. Diagnostischer Kit nach Anspruch 13, zusätzlich enthaltend Blocking-Puffer zur Sättigung freier Bindungsstellen der festen Phase, Waschpuffer und/oder Aprotinin.

18. Diagnostischer Kit nach Anspruch 13, wobei Plasmin in einer Pufferlösung gelöst oder lyophilisiert als Feststoff vorliegt.

19. Diagnostischer Kit nach Anspruch 13, wobei der Capture-Antikörper gegen ein Epitop des Prionproteins gerichtet ist, das sich im Bereich der Aminosäurereste 1-110 befindet, wenn der Detektionsantikörper gegen ein Epitop gerichtet ist, das außerhalb dieses Bereichs liegt, oder der Detektionsantikörper gegen ein Epitop gerichtet ist, das sich im Bereich der Aminosäurereste 1-110 befindet, wenn der Capture-Antikörper gegen ein Epitop gerichtet ist, das außerhalb dieses Bereichs liegt.

## Claims

1. Method for providing proof of pathological prion proteins *in-vitro* in a sample, whereby:
a) capture antibodies, which identify both the pathological (PrP^{Sc}) as well as the non-pathological form (PrP^{C}) of the prion protein, are fixed in a solid phase;
b) the sample with the fixed antibodies from step a) is incubated, whereby the pathological and the non-pathological form of the prion protein bond to the fixed antibodies with the formation of complexes;
c) the sample is separated from the derived complexes;
c) the complexes are incubated with plasmin, whereby the non-pathological form of the prion protein is split;
d) the split fragments obtained by the incubation with plasmin is separated from the complexes; and
f) the prion protein which is not split, obtained in the complexes, is detected with detection antibodies, which exhibit the capacity to bind PrP^{Sc} specifically.

2. Method according to claim 1, whereby the detection in step f) takes place quantitatively.

3. Method according to claim 1, whereby the detection antibodies identify both the pathological as well as the non-pathological form of the prion protein.

4. Method according to claim 1, whereby the capture antibody is directed against an epitope of the prion protein, which is located in the range of the amino acid residue 1-110 when the detection body is directed against an epitope, which lies outside this range, or the detection body is directed against an epitope which is located in the range of the amino acid residue 1-110 when the capture antibody is directed against an epitope which lies outside this range.

5. Method according to one of the foregoing claims, whereby, after step a), free binding points of the solid phase are saturated by blocking buffers.

6. Method according to one of the foregoing claims, whereby the solid phases are microtiter plates or paramagnetic or non-magnetic beads.

7. Method according to one of the foregoing claims, whereby the incubation period in step b) is not more than 2 hours.

8. Method according to one of the foregoing claims, whereby the incubation period in step b) is not more than 30 minutes.

9. Method according to one of the foregoing claims, whereby the addition of aprotinin follows the incubation with plasmin in step d).

10. Method according to one of the foregoing claims, whereby washing steps are carried out between the individual steps.

11. Method according to one of the foregoing claims, whereby the detection antibodies contain biotin, fluorescence markers and/or nanobeads, in particular nanobeads marked with europium.

12. Method according to one of the foregoing claims, whereby the detection in step f) takes place by means of an ELISA test.

13. Diagnostic kit for providing proof of pathological prion proteins *in-vitro* in a sample, containing:
a) capture antibodies, which are directed against both the pathological (PrP^{Sc}) as well as the non-pathological form (PrP^{C}) of the prion protein,
b) plasmin; and
c) detection antibodies which exhibit the capacity to bind PrP^{Sc} specifically.

14. Diagnostic kit according to claim 13, whereby the capture antibodies are already fixed on a solid phase,

15. Diagnostic kit according to claim 14, whereby the solid phase are microtiter plates or beads.

16. Diagnostic kit according to claim 13, whereby the detection antibodies identify both the pathological as well as the non-pathological form of the prion protein.

17. Diagnostic kit according to claim 13, additionally containing blocking buffers for the saturation of free binding points of the solid phase, wash buffers, and/or aprotinin.

18. Diagnostic kit according to claim 13, whereby plasmin is dissolved in a buffer solution or is present lyophilised as a solid.

19. Diagnostic kit according to claim 13, whereby the capture antibody is directed against an epitope of the prion protein which is located in the range of the amino acid residue 1-110 when the detection antibody is directed against an epitope which lies outside this range, or the detection antibody is directed against an epitope which is located in the range of the amino acid residue 1-110 when the capture antibody is directed against an epitope which lies outside this range.

## Revendications

1. Procédé pour déceler des protéines prions pathologiques in vitro dans un échantillon, ledit procédé comprenant les étapes consistant à :
a) fixer sur une phase solide des anticorps de capture qui reconnaissent aussi bien la forme pathologique (PrP^{Sc}) que la forme non patho-logique (PrP^{c}) de la protéine prion ;
b) incuber l'échantillon avec les anticorps fixés selon l'étape a), la forme pathologique et la forme non pathologique de la protéine prion se liant aux anticorps fixés en formant des complexes ;
c) détacher l'échantillon des complexes créés ;
d) incuber les complexes avec de la plasmine, la forme non pathologique de la protéine prion étant séparée ;
e) détacher des complexes les fragments séparés obtenus par l'incubation avec la plasmine ; et
f) déceler la protéine prion non séparée, contenue dans les complexes, à l'aide d'anticorps de détection qui ont la capacité de lier spécifiquement PrP^{Sc}.

2. Procédé selon la revendication 1, dans lequel la détection à l'étape f) s'effectue de façon quantitative.

3. Procédé selon la revendication 1, dans lequel les anticorps de détection reconnaissent aussi bien la forme pathologique que la forme non pathologique de la protéine prion.

4. Procédé selon la revendication 1, dans lequel l'anticorps de capture est dirigé contre un épitope de la protéine prion qui se trouve dans la zone des résidus d'acide aminé 1-110 lorsque l'anticorps de détection est dirigé contre un épitope qui se trouve en dehors de cette zone, ou l'anticorps de détection est dirigé contre un épitope qui se trouve dans la zone des résidus d'acide aminé 1-110, lorsque l'anticorps de capture est dirigé contre un épitope qui se trouve en dehors de cette zone.

5. Procédé selon l'une quelconque des revendications précédentes, dans lequel après l'étape a), des points de liaison libres de la phase solide sont saturés par des tampons de blocage.

6. Procédé selon l'une quelconque des revendications précédentes, dans lequel les phases solides sont des plaquettes de microtitration ou des billes paramagnétiques ou non magnétiques.

7. Procédé selon l'une quelconque des revendications précédentes, dans lequel le temps d'incubation à l'étape b) ne dure pas plus de 2 heures.

8. Procédé selon l'une quelconque des revendications précédentes, dans lequel le temps d'incubation à l'étape d) ne dure pas plus de 30 minutes.

9. Procédé selon l'une quelconque des revendications précédentes, dans lequel l'incubation avec la plasmine à l'étape d) est suivie d'une adjonction d'aprotinine.

10. Procédé selon l'une quelconque des revendications précédentes, dans lequel entre les étapes individuelles, des étapes de lavage sont effectuées.

11. Procédé selon l'une quelconque des revendications précédentes, dans lequel les anticorps de détection comprennent de la biotine, des marqueurs fluorescents et/ou des nanobilles, en particulier des nanobilles marquées avec de l'europium.

12. Procédé selon l'une quelconque des revendications précédentes, dans lequel la détection à l'étape f) est réalisée par un essai ELISA.

13. Kit de diagnostic pour déceler des prions pathologiques in vitro dans un échantillon, comprenant :
a) des anticorps de capture qui sont dirigés aussi bien contre la forme pathologique (PrP^{Sc}) que la forme non pathologique (PrP^{C}) de la protéine prion ;
b) de la plasmine ; et
c) des anticorps de détection ayant la capacité de lier spécifiquement Prp^{Sc}.

14. Kit de diagnostic selon la revendication 13, dans lequel les anticorps de capture sont déjà fixés sur une phase solide.

15. Kit de diagnostic selon la revendication 14, dans lequel la phase solide correspond à des plaquettes de microtitration ou à des billes.

16. Kit de diagnostic selon la revendication 13, dans lequel les anticorps de détection reconnaissent aussi bien la forme pathologique que non pathologique de la protéine prion.

17. Kit de diagnostic selon la revendication 13, comprenant en outre un tampon de blocage pour saturer des points de liaison libres de la phase solide, des tampons de lavage et/ou de l'aprotinine.

18. Kit de diagnostic selon la revendication 13, dans lequel la plasmine est présente sous forme dissoute dans une solution tampon ou sous forme lyophilisée en tant que matière solide.

19. Kit de diagnostic selon la revendication 13, dans lequel les anticorps de capture sont dirigés contre un épitope de la protéine prion qui se trouve dans la zone des résidus d'acide aminé 1-110, lorsque l'anticorps de détection est dirigé contre un épitope qui se trouve en dehors de cette zone, ou l'anticorps de détection est dirigé contre un épitope qui se trouve dans la zone des résidus d'acide aminé 1-110, lorsque l'anticorps de capture est dirigé contre un épitope qui se trouve en dehors de cette zone.
